(19) Europäisches Patentamt
European Patent Office
Office européen des brevets

(11) **EP 3 511 715 A1**

(12) **EUROPEAN PATENT APPLICATION**

(43) Date of publication:
**17.07.2019 Bulletin 2019/29**

(51) Int Cl.:
***G01N 33/68*** (2006.01)

(21) Application number: **18151827.5**

(22) Date of filing: **16.01.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Koninklijke Philips N.V.**
**5656 AE Eindhoven (NL)**

(72) Inventors:
- **KOOIJMAN, Gerben**
  **5656 AE Eindhoven (NL)**
- **RMAILE, Amir Hussein**
  **5656 AE Eindhoven (NL)**
- **GLASSE, Carl**
  **5656 AE Eindhoven (NL)**

- **DE JAGER, Marinus Karel Johannes**
  **5656 AE Eindhoven (NL)**
- **VAN HARTSKAMP, Michael Alex**
  **5656 AE Eindhoven (NL)**
- **CHAPPLE, Iain Leslie Campbell**
  **5656 AE Eindhoven (NL)**
- **GRANT, Melissa Mackay**
  **5656 AE Eindhoven (NL)**
- **PRESHAW, Philip**
  **5656 AE Eindhoven (NL)**
- **TAYLOR, John J.**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **PERIODONTAL DISEASE DIAGNOSTIC METHODS, USES AND KITS**

(57) Disclosed is an *in vitro* method for assessing whether a human patient is free from periodontal disease, has gingivitis, has mild periodontitis or has advanced periodontitis. The method is based on the insight to determine biomarker proteins. Accordingly, in a sample of saliva of a patient, the concentrations are measured of the proteins: Alpha-1-acid glycoprotein (A1AGP) and Pyruvate Kinase (PK), and at least one of the proteins Matrix metalloproteinase-9 (MMP-9) and S100 calcium binding protein A8 (S100A8). Based on the concentrations as measured, at least one value is determined reflecting the joint concentrations for said proteins. This at least one value may indicate the probability that human patient is free from periodontal disease, has gingivitis, has mild periodontitis or has advanced periodontitis. The at least one value can be compared with at least one threshold value reflecting in the same manner the joint concentrations associated with gingivitis, mild or advanced periodontitis. The comparison allows assessing whether the testing value is indicative of the periodontal health status in said patient.

FIG. 1

**Description**

FIELD OF THE INVENTION

The invention is in the field of oral care, and pertains to saliva-based diagnostics of periodontal disease. Particularly, the invention pertains to a kit, use and methods for distinguishing between states of healthy gums, gingivitis, mild periodontitis and advanced periodontitis.

BACKGROUND OF THE INVENTION

Gum inflammation, or gingivitis, is a non-destructive periodontal disease caused mainly by the adherence of dental bacterial biofilms, or dental plaque, to the tooth surface. If not detected and treated, the reversible gingivitis usually leads to the inflammation of the tissues surrounding the tooth (i.e. periodontal tissues), a condition defined as periodontitis, which is irreversible and causes tissue destruction and alveolar bone loss, and ultimately results in the loss of teeth. During the progression of gum disease, there are usually clinical signs and symptoms associated with it, such as the swelling of the gums, the change in color from pink to dark red, the bleeding of the gums, bad breath, and the gums becoming more tender or painful to touch.

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.

Early and accurate diagnosis of periodontal disease, thus, is important from both an oral and overall health perspective.

Periodontal diseases are still poorly diagnosed in general dental practice, resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease. Hence, more objective, faster, accurate, easier-to-use diagnostics which preferably may also be performed by non-specialists are desirable. Thereby it is desirable to measure current disease activity, and possibly a subject's susceptibility to further periodontal disease.

Saliva or oral fluids have long been advocated as a diagnostic fluid for oral and general diseases, and with the advent of miniaturized biosensors, also referred to as lab-on-a-chip, point of care diagnostics for rapid chair-side testing have gained greater scientific and clinical interest. Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented. For example, Ramseier et al (J Periodontol. 2009 Mar;80(3):436-46) identified host- and bacterially derived biomarkers correlated with periodontal disease. However, no definite test has emerged yet.

Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.

Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.

Moreover, periodontitis can manifest itself across the entire spectrum of severity ranging from mild to advanced forms of the disease. In order to easily assess the severity of the condition, dentists often classify patients suffering from periodontitis into two groups - those suffering from mild periodontitis, and those suffering from *advanced* periodontitis. The available methods of making such an assessment, however, involve a labor intensive process that a dentist will not perform routinely on every patient and/or on every visit, and that is impossible to perform by a consumer (self-diagnosis).

It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that the patient is to be classified as having a healthy mouth, or suffering from gingivitis, mild periodontitis or advanced periodontitis.

SUMMARY OF THE INVENTION

In order to better address the foregoing desires, the invention, in one aspect, concerns an *in vitro* method for assessing whether a human patient is free from periodontal disease, has gingivitis, has mild periodontitis or has advanced periodontitis, the method comprising detecting, in a sample of saliva from said human patient, the concentrations of the proteins: Alpha-1-acid glycoprotein (A1AGP) and Pyruvate Kinase (PK), and at least one of the proteins Matrix metalloproteinase-9 (MMP-9) and S100 calcium binding protein A8 (S100A8); determining at least one testing value reflecting the joint concentrations determined for said proteins; and assessing whether the at least one testing value is indicative for freedom from periodontal disease, gingivitis, mild periodontitis or advanced periodontitis in said patient.

In another aspect, the invention presents the use of the proteins Alpha-1-acid glycoprotein (A1AGP) and Pyruvate Kinase (PK), and at least one of the proteins Matrix metalloproteinase-9 (MMP-9) and S100 calcium binding protein A8 (S100A8) in a saliva sample of a human patient, as biomarkers for assessing whether the patient is free from periodontal disease, has gingivitis, has mild periodontitis or has advanced periodontitis. Optionally, the age of the patient is also used as a biomarker.

In a further aspect, the invention resides in a system assessing whether a human patient is free from periodontal disease, has gingivitis, has mild periodontitis or has advanced periodontitis, the system comprising:

detection means able and adapted to detect in a sample of saliva of the human patient the proteins Alpha-1-acid glycoprotein (A1AGP) and Pyruvate Kinase (PK), and at least one of the proteins Matrix metalloproteinase-9 (MMP-9) and S100 calcium binding protein A8 (S100A8); and

a processor able and adapted to determine from the determined concentrations of said proteins an indication whether the patient is free from periodontal disease, has gingivitis, has mild periodontitis or has advanced periodontitis.

The system optionally contains a data connection to an interface, particularly a graphical user interface, capable of presenting information, preferably also capable of putting in information, said interface being either a part of the system or a remote interface.

Optionally one or more of the foregoing items, particularly the processor, are enabled to function "in the cloud", i.e., not on a fixed machine, but by means of an internet-based application.

In a still further aspect, the invention provides a kit for detecting at least three biomarkers for periodontal disease in a sample of saliva of a human patient, said kit comprising one or more, typically three, detection reagents for detecting Alpha-1-acid glycoprotein (A1AGP), Pyruvate Kinase (PK), and at least one of the proteins Matrix metalloproteinase-9 (MMP-9) and S100 calcium binding protein A8 (S100A8). Typically, three or more detection reagents are used, each of which binds a different biomarker. In one embodiment, a first detection reagent is capable of binding A1AGP, a second detection reagent is capable of binding PK, and a third detection reagent is capable of binding one of S100A8 and MMP-9.

In yet another aspect, the invention provides an *in vitro* method for determining a change in status of periodontal disease in a human patient over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins: A1AGP, PK, and at least one of S100A8 and MMP-9, and comparing the concentrations, whereby a difference in any one, two or all three of the concentrations, reflects a change in status.

In a further aspect, the invention provides a method of diagnosing whether a human patient is free from periodontal disease, has gingivitis, has mild periodontitis or has advanced periodontitis, comprising detecting in a sample of saliva of the human patient the proteins Alpha-1-acid glycoprotein (A1AGP) and Pyruvate Kinase (PK), and at least one of the proteins Matrix metalloproteinase-9 (MMP-9) and S100 calcium binding protein A8 (S1 00A8), and assessing the presence or absence of gingivitis, mild periodontitis or advanced periodontitis in the patient on the basis of the concentrations of said proteins in said sample. Optionally, the method of this aspect comprises the further step of treating the periodontitis in the patient.

In yet a further aspect, the invention provides a method of detecting the proteins Alpha-1-acid glycoprotein (A1AGP) and Pyruvate Kinase (PK), and at least one of the proteins Matrix metalloproteinase-9 (MMP-9) and S100 calcium binding protein A8 (S100A8) in a human patient suffering from gingivitis, mild or advanced periodontitis, comprising:

(a) obtaining a saliva sample from a human patient; and
(b) detecting whether Alpha-1-acid glycoprotein (A1AGP) and Pyruvate Kinase (PK), and at least one of the proteins Matrix metalloproteinase-9 (MMP-9) and S100 calcium binding protein A8 (S100A8) are present in the sample by contacting the sample with one or more detecting reagents for binding said proteins and detecting binding between each protein and the one or more detecting reagents. Typically, there is a first detection reagent capable of binding A1AGP, a second detection reagent is capable of binding PK, and a third detection reagent is capable of binding one of MMP-9 and S100A8.

BRIEF DESCRIPTION OF THE DRAWINGS

Fig. 1 schematically represents a system for use in the method as described in this disclosure.
Fig. 2 is an example of a classification scheme for the stages of periodontal diseases.
Fig. 3 is an example of a decision tree approach to the classification of periodontal health.

DETAILED DESCRIPTION OF THE INVENTION

In a general sense, the invention is based on the judicious insight that as few as three proteins can serve as biomarkers in a sample of saliva of a human patient, for classifying the periodontal disease status of the patient as being in one of four categories: a first category being the absence of periodontal disease; a second category being gingivitis; a third category being mild or moderate periodontitis (i.e., not advanced, hereinbefore and hereinafter the term "mild periodontitis" will include moderate periodontitis, unless indicated otherwise); and a fourth category being advanced periodontitis.

The third category is hereinafter collectively indicated as mild periodontitis.

The invention can therefore distinguish between: a healthy mouth requiring no treatment for periodontal disease; gingivitis, allowing for timely intervention to reverse its effects before it proceeds to periodontitis; mild periodontitis, indicating a need for treatment; or advanced periodontitis, indicating an acute need for significant treatment.

The biomarker proteins are Alpha-1-acid glycoprotein (A1AGP), Pyruvate Kinase (PK), and either or both of Matrix metalloproteinase-9 (MMP-9) and S100 calcium binding protein A8 (S100A8). The subject's age may optionally be included as an additional marker.

Alpha-1-acid glycoprotein (A1AGP) is a plasma alpha-globulin glycoprotein synthesized primarily by the liver. It is also sometimes known as Orosomucoid. It functions as a transport protein in the blood acts as a carrier of basic and neutrally charged lipohillic compounds. It is also believed to regulate the interaction between blood cells and endothelial cells.

Pyruvate kinase catalyses the final step of glycolysis. There are four tissue-specific isozymes of Pyruvate Kinase, each having particular kinetic properties needed for different tissues.

S100 calcium binding protein A8 is a calcium- and zinc-binding protein which plays a prominent role in the regulation of inflammatory processes and immune response. It can induce neutrophil chemotaxis and adhesion.

MMPs are a family of enzymes that are responsible for the degradation of extracellular matrix components such as collagen, proteoglycans, laminin, elastin, and fibronectin. They play a central role in the periodontal ligament (PDL) remodelling, both in physiological and pathological conditions. MMP-9, also known as 92 kDa type IV collagenase, 92 kDa gelatinase or gelatinase B (GELB), is a matrixin, a class of enzymes that belong to the zinc-metalloproteinases family involved in the degradation of the extracellular matrix.

The four proteins mentioned above are known in the art. The skilled person is aware of their structure, and of methods to detect them in an aqueous sample, such as a saliva sample. Hereinafter the aforementioned protein biomarkers are collectively referred to as "the biomarker panels of the invention." A biomarker panel of the invention, in one embodiment, may consist of the four protein biomarkers identified in the invention, i.e. A1AGP, PK, MMP-9 and S100A8. Preferably, a biomarker panel of the invention consists of not more than three of the protein biomarkers identified in the invention, i.e., A1AGP, PK and MMP-9; or A1AGP, PK and S100A8. In addition to the biomarker panels of the invention, other biomarkers and or data, such as demographic data (e.g., age, sex) can be included in a set of data applied for the determination of the type of periodontitis. Additional biomarkers include one or more of the proteins Matrix metalloproteinase-8 (MMP-8), Interleukin-1β, Free Light Chain Kappa, Free Light Chain Lambda, Haemoglobin beta, Haemoglobin delta, and Hepatocyte Growth Factor (HGF).

Preferred extended biomarker panels are:

A1AGP + PK + MMP-9 + MMP-8
A1AGP + PK + MMP-9 + IL-1β
A1AGP + PK + MMP-9 + Free Light Chain Kappa
A1AGP + PK + MMP-9 + Free Light Chain Lambda
A1AGP + PK + MMP-9 + Haemoglobin beta
A1AGP + PK + MMP-9 + Haemoglobin delta

A1AGP + PK + S100A8 + MMP-8
A1AGP + PK + S100A8 + Hepatocyte Growth Factor
A1AGP + PK + S100A8 + IL-1β

When other biomarkers are optionally included, the total number of biomarkers (i.e. the biomarker panel of the invention plus other biomarkers) is typically 4, 5 or 6.

However, a desirable advantage of the present invention is that the classification of periodontal disease in a patient can be determined by measuring preferably not more than four biomarkers, and more preferably measuring only three biomarkers, with the biomarker panel A1AGP, PK, and either of MMP-9 or S100A8 being preferred. Particularly, the determination does not need to involve the use of other data, which advantageously provides a simple and straightforward diagnostic test.

The method, as desired, requires only that a small saliva sample, e.g. a dropsize, is taken from the subject. The size of the sample will typically range of from 0.1 μl to 2 ml, such as 1-2 ml, whereby smaller amounts, e.g., 0. 1 to 100 μl can be used for *in vitro* device processing, and whereby taking a larger sample, such as up to 20 ml, such as 7.5 to 17 ml, is also possible.

This sample is entered into an *in vitro* diagnostic device, which measures the concentrations of the at least three proteins involved, and which returns a diagnostic outcome, classifying the subject on the basis of a likelihood of having no periodontal disease, having gingivitis, having mild periodontitis or having advanced periodontitis.

The ease of use of this invention will make it possible to test the majority of dental patients, or those with a high risk for developing periodontal disease, on a regular basis (e.g. as part of a regular dental check or even at home). This allows, *inter alia,* detecting the presence of gingivitis, or detecting the presence of mild periodontitis before it proceeds to advanced periodontitis, and thus enables more timely taking oral care measures to prevent periodontitis from developing or advancing. Or, e.g., with patients known to be at high risk for periodontitis, and tested for the first time, the method allows to identify whether the periodontitis is mild or advanced. Also, the method can be applied after treatment of a patient previously diagnosed with advanced periodontitis, in order to check whether the periodontitis has improved so as to become mild. Similarly, the method can be applied after treatment of a patient previously diagnosed with mild periodontitis, in order to check whether the periodontal disease has improved so as to become gingivitis. The ability to assess that the patient is "healthy", i.e. free from the periodontal diseases gingivitis, mild and advanced periodontitis, is beneficial to confirm, for example, that the patient's current oral health care routine is satisfactory. Particularly, the method is also suitable for self-diagnosis, whereby the steps of taking the sample and entering it into a device can be conducted by the patient him- or herself.

The patient may typically be known to have periodontal disease when the invention is carried out. The patient may typically be known to have periodontitis when the invention is carried out to determine whether the periodontitis is mild or advanced. In certain embodiments therefore, the method is for assessing whether a human patient, known to have periodontitis, has mild periodontitis or advanced periodontitis.

A method of the invention typically comprises detecting the aforementioned at least three proteins making up a biomarker panel of the invention, and optional further biomarker proteins, by using one or more detection reagents.

The "saliva" that is tested according to the invention may be undiluted saliva, which may be obtained by spitting or swabbing, or diluted saliva, which may be obtained by rinsing the mouth with a fluid. Diluted saliva may be obtained by the patient rinsing or swilling their mouth for a few seconds with sterile water (for example 5ml or 10ml) or other suitable fluid, and spitting into a container. Diluted saliva may sometimes be referred to as an oral rinse fluid.

By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

The term "concentration" with respect to the protein biomarkers is to be given its usual meaning, namely the abundance of the protein in a volume. Protein concentration is typically measured in mass per volume, most typically mg/ml or μg/ml, but sometimes as low as pg/ml. An alternative measure is Molarity (or Molar concentration), mol/L or "M". The concentration can be determined by detecting the amount of protein in a sample of known, determined or pre-determined volume.

An alternative to determining the concentration is to determine the absolute amount of the protein biomarker in the sample, or determining the mass-fraction of the biomarker in the sample, for example the amount of the biomarker relative to the total of all other proteins in the sample.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The term "periodontal disease" refers to gingivitis and periodontitis (mild and advanced). A patient that is free from periodontal disease is said to be healthy.

The phrase "specifically (or selectively) binds" or "specifically (or selectively) immunoreactive with," when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (*see, e.g.,* Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region. The antibody may be a bispecific antibody, e.g. an antibody that has a first variable region that specifically binds to a first antigen and a second variable region that specifically binds to a second, different, antigen. Use of at least one bispecific antibody can reduce the number of detection reagents needed.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. Specificity may also be referred to as the true negative rate.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaCl, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 1251) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

Since multiple markers are used, a threshold can be determined on the basis of the joint concentrations of these biomarkers. This threshold determines whether a patient is classified as free from periodontal disease, has gingivitis, has mild periodontitis or has advanced periodontitis. The invention reflects the insight that all stages of periodontal health, from healthy through to advanced periodontitis, can be detected with sufficient accuracy based on a measurement of the combination of biomarkers as indicated above.

This insight supports another aspect, the invention, which is the use of the proteins A1AGP, PK, and at least one of MMP-9 and S100A8, as biomarkers in a saliva sample of a human patient, as biomarkers for assessing whether the patient is free from periodontal disease, has gingivitis, has mild periodontitis or has advanced periodontitis.

This use can be implemented in a method as substantially described hereinbefore and hereinafter.

The method of the invention comprises determining at least one testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power. Optionally, the testing value reflects the joint concentrations determined for said proteins in combination with the age of the subject. In certain embodiments, a testing value can be generated for one, two or three of (i) the probability of periodontitis versus health or gingivitis, (ii) the probability of gingivitis versus health, and (iii) the probability of advanced periodontitis versus mild periodontitis.

The resulting joint concentration value may be compared with one or more threshold values reflecting in the same manner the joint concentrations associated with the presence of periodontitis, the presence of advanced periodontitis or the presence of gingivitis. The comparison allows assessing whether the testing value is indicative of the presence of a healthy mouth, gingivitis, mild periodontitis or advanced periodontitis in the patients whose saliva is subjected to the test.

The threshold value can, e.g., be the joint concentration value, obtained in the same manner on the basis of the concentrations determined for the same proteins in a reference sample associated with the presence of advanced periodontitis, i.e. in a patient diagnosed with advanced periodontitis. Typically, thereby a value reflecting the same or higher joint concentration is indicative of the presence of advanced periodontitis in a tested patient. Analogously, a value reflecting a lower joint concentration in the saliva of a tested periodontitis patient, indicates that the periodontitis is mild or moderate (i.e., not advanced). However, it will be understood that it is also possible to calculate a threshold value (e.g. by using a negative multiplier) such that a testing value indicating advanced periodontitis would be below the threshold, and a testing value indicating mild periodontitis, would be above the threshold.

The threshold value can also be determined on the basis of measuring the concentrations of the present biomarker proteins in a set of samples, including patients with a known diagnosis of gingivitis, periodontitis (optionally mild/moderate only) and advanced periodontitis. Thereby the measured concentration values can be subjected to statistical analysis, possibly including machine learning methods, allowing to discriminate, with the desired sensitivity and specificity, patients classified as healthy, as having gingivitis, as having mild or moderate periodontitis and as having advanced periodontitis. Therefrom, the desired threshold value(s) can be obtained. On the basis of this threshold value, a sample to be tested can be subjected to the same concentration measurement, and the concentration values are then processed, in the same manner in which the threshold value(s) is obtained, so as to determine a joint concentration value that can be compared with the threshold, thus allowing the tested sample to be classified as having mild or advanced periodontitis.

In an interesting embodiment, the joint concentration value is obtained in the form of a score as follows. A numerical value (protein concentration values in e.g. ng/ml) is assigned to each measurement, and these values are used in a linear or non-linear combination to calculate a score between zero and one. In the event that the threshold value is determined on the basis of a set of subjects as mentioned above, the score between 0 and 1 is typically calculated with the sigmoid function that takes the joint concentration as input (as shown further on). For example, based on the protein concentrations, three scores (between 0 and 1) can be calculated:

$S_P$: this can be considered to be the probability of periodontitis 'versus' health/ gingivitis

$S_G$: this can be considered to be the probability of gingivitis 'versus' health

$S_{AP}$: this can be considered to be the probability of advanced periodontitis 'versus' mild periodontitis

The probabilities (between 0 and 1) of the four disease stages may then be calculated as follows:

| | |
|---|---|
| Health (H): | $(1-S_P)\cdot(1-S_G)$ |
| Gingivitis (G): | $(1-S_P)\cdot S_G$ |
| Mild Periodontitis (MP) : | $S_P\cdot(1-S_{AP})$ |
| Advanced Periodontitis (AP): | $S_P\cdot(S_{AP})$ |

The threshold may be chosen based on the desired sensitivity and specificity. It will be understood by those in the art that the probabilities set out above should be considered as an idealized simplification and that different estimates may be used in practice taking probabilistic dependency into account.

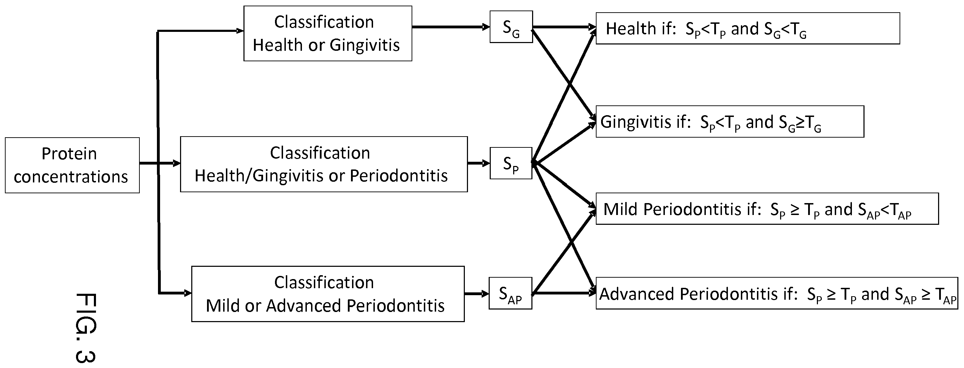

Alternatively, a decision-tree like approach may be taken, where first a classification on health or gingivitis versus periodontitis is made: a patient is classified as having periodontitis when $S_P \geq T_P$, with $T_P$ a threshold. Subsequently, further refinement is made. A periodontitis classified patient is further classified as having advanced periodontitis when $S_{AP} \geq T_{AP}$, with $T_{AP}$ a threshold. Otherwise the classification is mild periodontitis. A patient classified as health or gingivitis is further classified as having gingivitis when $S_G \geq T_G$, with TG a threshold, otherwise the patient is classified as healthy. The thresholds $T_P, T_{AP}, T_G$ may be chosen according to the desired tuning of the sensitivity and specificity of the respective classification steps. This decision tree approach is depicted in Fig. 3.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

0 = absence of inflammation,

1 = mild inflammation; slight change in color little change in texture of any portion of but not the entire margin or papillary gingival unit,

2 = mild inflammation; but involving entire margin or papillary unit,

3 = moderate inflammation; glazing, redness, oedema and/or hypertrophy of margin or papillary unit,

4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.

The resulting subject group (patient group) definition is as follows:

Healthy group (H): PD $\leq$ 3 mm in all sites (but would allow up to four 4 mm pockets at distal of last standing molars), no sites with interproximal attachment loss, GI of $\geq$ 2.0 in $\leq$ 10% sites, %BOP scores $\leq$ 10%;

Gingivitis group (G): GI $\geq$ 3.0 in > 30% of sites, no sites with interproximal attachment loss, no sites with PD > 4 mm , %BOP scores > 10%;

Mild-moderate periodontitis group (MP): interproximal PD of 5-7 mm, (equating to approximately 2-4 mm CAL) at $\geq$ 8 teeth, %BOP scores > 30%;

Advanced periodontitis group (AP): interproximal PD of $\geq$ 7 mm, (equating to approximately $\geq$ 5 mm CAL) at $\geq$ 12 teeth, %BOP scores > 30%.

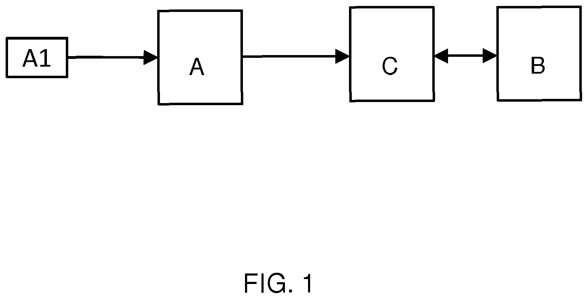

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).

As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.

The measurement device (A) is configured to receive a saliva sample, for example by putting a drop of saliva on a cartridge (A1), which can be inserted into the device (A). The device can be an existing device that is capable to determine, from the same saliva sample, the concentrations of at least the proteins A1AGP, PK, and at least one of the proteins S100A8 and MMP-9.

The measurement device (A) should be able to receive a saliva sample, for example by putting a drop of saliva on a cartridge (A1), which can be inserted into the device (A). The device may be an existing device that is capable to determine, from the same saliva sample, the concentrations of at least the proteins A1AGP, PK, and one of MMP-9 and S100A8.

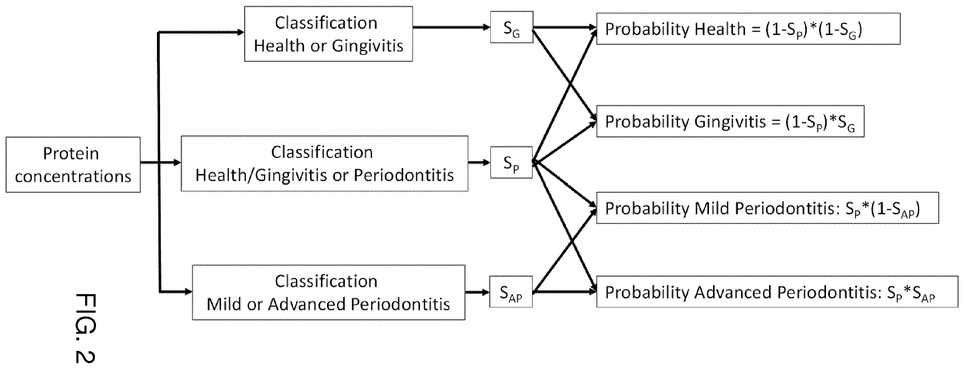

The processing unit (C) receives numerical values for the protein concentrations from part (A). The unit (C) is provided with software (typically embedded software) allowing it to classify the disease stage. For example, the classification may be as indicated in Fig. 2. Based on the protein concentrations, three scores (between 0 and 1) can be calculated:

$S_P$: this can be considered to be the probability of periodontitis 'versus' health/gingivitis
$S_G$: this can be considered to be the probability of gingivitis 'versus' health
$S_{AP}$: this can be considered to be the probability of advanced periodontitis 'versus' mild periodontitis

The probabilities (between 0 and 1) of the four disease stages may then be calculated as follows (noting, as above, that different estimates may be used in practice taking probabilistic dependency into account):

| | |
|---|---|
| Health (H): | $(1-S_P)\cdot(1-S_G)$ |
| Gingivitis (G): | $(1-S_P)\cdot S_G$ |
| Mild Periodontitis (MP) : | $S_P\cdot(1-S_{AP})$ |
| Advanced Periodontitis (AP): | $S_P\cdot(S_{AP})$ |

The unit (C) can output these probabilities directly to unit (B), for example a probability for health of 0.8 would indicate health with 80% certainty. Alternatively unit (C) can output an indication (I) of the disease state to the GUI (B), the indicated disease state would be the one with the highest probability.

In another embodiment, the software may further include numerical values for thresholds ($T_P$, $T_G$, $T_{AP}$). Then, for instance, if the calculated score $S_P$ exceeds $T_P$, the subject is considered to have periodontitis (thus $S_P :=1$ for $S_P \geq T_P$ and $S_P :=0$ for $S_P <T_P$). Further refinement between mild and advanced periodontitis is then made on basis of $S_{AP}$. For instance, indication 'advanced periodontitis' if $S_{AP} \geq 0.5$, or alternatively indication 'advanced periodontitis' if $S_{AP}$ exceeds threshold $T_{AP}$. The use of specific thresholds allows to tune the sensitivity and specificity of classification of the disease states to a desired level. For instance, applying a lower threshold $T_P$ for periodontitis increases sensitivity for detecting periodontitis (fewer false negatives) at cost of lower specificity (more false positives).

Certainty of indication may also be achieved by definition of (a) range(s) R1-R2 or (general) thresholds T. For instance, if R1<$S_P$<R2, periodontitis classification may be considered as inconclusive, and no further classification is made. The indication (I) will then read 'inconclusive'. Or, if neither of the calculated probabilities for health, gingivitis, mild periodontitis, and advanced periodontitis exceeds the threshold T, indication (I) will read 'inconclusive'

A specific calculation of the scores can be implemented, e.g., by means of a sigmoid function applying the following formula:

$$S = \frac{1}{1 + \exp(-(c_0 + \sum\limits_{i=1}^{N} c_i B_i))}$$

wherein $N$ is the number of proteins/biomarkers used. $c_0$, $c_1$, etc. are coefficients (numerical values) and $B_1$, $B_2$, etc. are the respective protein concentrations.

Determining of the coefficients can be done by a training procedure for each of the three classification steps (H/G vs P, resp. H vs G, resp. MP vs AP):

Select $N_P$, respectively, No, respectively $N_{AP}$ subjects with periodontitis respectively gingivitis, respectively advanced periodontitis (as identified by a dentist via the current criteria) and $N_{NP}$, $N_H$, $N_{AP}$ subjects without periodontitis, respectively with healthy gums, respectively with mild periodontitis.

Note that for subjects being part of one study we have $N_P = N_{MP} + N_{AP}$, $N_{NP} = N_H + N_G$.

Take a saliva sample from each subject and determine the protein concentrations of a combination of biomarkers as explained above.

Define the score $S_P$, respectively $S_G$, respectively $S_{AP}$ to be 1 for periodontitis, respectively gingivitis, respectively advanced periodontitis and to be 0 for no periodontitis, respectively healthy gums, respectively mild periodontitis.

Fit the sigmoid function to the scores and protein concentration values.

Other regression or machine learning methods (linear regression, neural network, support vector machine) may be used to train a classifier that predicts oral health status based on the protein concentrations, where the scores $S_P$, $S_G$, $S_{AP}$ are high for the periodontitis, respectively gingivitis, respectively advanced periodontitis patients and low for the non-periodontitis, respectively healthy, respectively mild periodontitis controls.

In particular, such a procedure has been applied using a clinical study with subjects having either a healthy oral conditions, gingivitis, mild periodontitis, or advanced periodontitis (identified by clinical assessment by a dental professional via current criteria, e.g. American Academy of Periodontology criteria). Performance of various biomarker combinations were evaluated by means of logistic regression with leave-one-out cross validation (LOOCV), resulting in the preferred biomarker combinations of the invention.

With reference to the aforementioned system, the invention also provides, in a further aspect, a system for assessing whether a human patient is free from periodontal disease, has gingivitis, has mild periodontitis or has advanced periodontitis, the system comprising:

detection means able and adapted to detect in a sample of saliva of the human patient the proteins A1AGP, PK, and at least one of MMP-9 and S100A8; As explained above, such means are known, and easily accessible to the skilled person; Typically, there is provided a container for receiving an oral sample of a subject therein, the container provided with the detection means;
a processor able and adapted to determine from the determined concentrations of said proteins an indication of the patient having no periodontal disease, having gingivitis, having mild periodontitis or having advanced periodontitis.

Optionally, the system comprises a user interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information, such as, e.g., the age of the subject, sex, BMI (Body Mass Index).

The invention also provides, either separately or as part of the aforementioned system, a kit for detecting at least three biomarkers for periodontal disease in a sample of saliva of a human patient, said kit comprising one or more detection reagents for detecting A1AGP, PK, and one or both of S100A8 and MMP-9. Typically, the kit comprises three detection reagents, each directed to a different biomarker, wherein a first detection reagent is capable of binding A1AGP, a second detection reagent is capable of binding PK, and a third detection reagent is capable of binding one or both of S100A8 and MMP-9. As discussed above with reference to the method of the invention, the kit may comprise more detection reagents, such as particularly for MMP-8, Interleukin-1β, Haemoglobin-beta, Haemoglobin-delta, Hepatocyte Growth Factor, Free Light Chain kappa or Free Light Chain lambda, and/or for other proteins. In a preferred embodiment the detection reagents made available in the kit consist of the detection reagents for the selection of three proteins making up a 3-biomarker panel of the invention, as mentioned.

Preferably said kits comprise a solid support, such as a chip, a microtiter plate or a bead or resin comprising said detection reagents. In some embodiments, the kits comprise mass spectrometry probes, such as ProteinChip™.

The kits may also provide washing solutions and/or detection reagents specific for either unbound detection reagent or for said biomarkers (sandwich type assay).

In an interesting aspect, the recognition of a biomarker panel of the invention is applied in monitoring the status of periodontal disease in a human patient, over time. Accordingly, the invention also provides an *in vitro* method for determining a change in status of periodontal disease in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins: A1AGP, PK, and one or both of S100A8 and MMP-9, and comparing the concentrations, whereby a difference of preferably at least two, and more preferably all three concentrations, reflects a change in status. This difference can be reviewed as a difference in concentrations, thus allowing a direct comparison without first generating a number between 0 and 1, or any other classification. It will be understood that the measurements received at both points in time can also be processed in just the same manner as done when determining the patient status as above.

The invention also provides a method of diagnosing whether a human patient is free from periodontal disease, has gingivitis, has mild periodontitis or has advanced periodontitis, comprising detecting in the patient's saliva the presence of the proteins A1AGP, PK, and one or both of S100A8 and MMP-9. The presence of healthy mouth, gingivitis, mild periodontitis or advanced periodontitis in the patient is assessed on the basis of the concentrations of said proteins in said sample. Optionally, the method of this aspect comprises the further step of treating the periodontal disease in the patient. This optional treatment step can comprise the administration of known therapeutic agents or dental procedures, or a combination of therapeutic agents and dental procedures. Known therapeutic agents include the administration of antimicrobial-containing agents such as a mouthwash, chip, gel or microsphere. A typical antimicrobial agent for use in treating gingivitis and periodontitis is chlorhexidine. Other therapeutic agents include antibiotics, typically orally-administered antibiotics, and enzyme suppressants such as doxycycline. Known non-surgical therapeutic procedures include scaling and root planing (SRP). Known surgical procedures include surgical pocket reduction, flap surgery, gum grafts or bone grafts.

The invention further provides a method of detecting the A1AGP, PK, and one or both of S100A8 and MMP-9 in a patient suffering from gingivitis, mild or advanced periodontitis, comprising:

(a) obtaining a saliva sample from a human patient; and
(b) detecting whether A1AGP, PK, and one or both of S100A8 and MMP-9 are present in the saliva sample by contacting the saliva sample with a first detection reagent capable of binding A1AGP, a second detection reagent is capable of binding PK, and a third detection reagent is capable of binding one of S100A8 and MMP-9 and detecting binding between each protein and detection reagent.

The invention will be further illustrated with reference to the following nonlimiting example.

Example:

In a clinical study with 153 subjects, of whom 39 had healthy gums (H), 35 were diagnosed with gingivitis (G), 41 were diagnosed with mild periodontitis (MP), and 38 were diagnosed with advanced periodontitis (AP), Receiver-Operator-Characteristic Area-Under-the Curve values were obtained. ROC AUC values of >0.9 were obtained for detecting periodontitis, and values >0.75 for subsequently distinguishing health from gingivitis and distinguishing mild from advanced periodontitis.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting the cumulative distribution function (area under the probability distribution from - ∞ to the discrimination threshold) of the detection probability on the y-axis, versus the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

$$0.90\text{-}1 = \text{excellent (A)}$$

$$0.80\text{-}0.90 = \text{good (B)}$$

$$0.70\text{-}0.80 = \text{fair (C)}$$

$$0.60\text{-}0.70 = \text{poor (D)}$$

$$0.50\text{-}0.60 = \text{fail (F)}$$

Various biomarker combinations were evaluated by means of logistic regression with leave-one-out cross validation (LOOCV), resulting in the biomarker combinations of the invention. It can be seen that the protein biomarker combinations of the invention have an AUC of >0.75 for all three classifications (Healthy/Gingivitis v Periodontitis, Healthy vs Gingivitis, and Mild Periodontitis vs, Advanced Periodontitis).

Based on the foregoing, in the results of the aforementioned clinical study, an ROC AUC value of above 0.75 is considered to represent a desirable accuracy for providing a diagnostic test in accordance with the invention.

Table 1 below summarizes, from among all biomarkers and biomarker panels determined, the data representing an ROC AUC value of above 0.75.

The biomarker proteins presented in the table are Alpha-1-acid glycoprotein (A1AGP) and Pyruvate Kinase (PK), Matrix metalloproteinase-9 (MMP-9) and S100 calcium binding protein A8 (S100A8), as well as MMP-8, Interleukin-1β, Haemoglobin-beta, Haemoglobin-delta, Hepatocyte Growth Factor, Free Light Chain kappa and Free Light Chain lambda.

It has now been found that the optimal results, of having a ROC AUC above 0.75 applying as few markers as possible, is contingent on the selection of a biomarker panel consisting of Alpha-1-acid glycoprotein (A1AGP), Pyruvate Kinase (PK), and one or both of Matrix metalloproteinase-9 (MMP-9) and S100 calcium binding protein A8 (S100A8), or with further markers added as shown.

<u>Table 1</u>

| MMP8 | IL1B | MMP9 | HGF | Age | κ | λ | κ+λ | κ/λ | κ-λ | A1AGP | Hb-beta | Hb-delta | Keratin 4 | Profilin | Pyruvate Kinase | S100A8 | S100A9 | HG vs P AUC LOOCV | H vs G AUC LOOCV | MP vs AP AUC LOOCV |
|---|---|---|---|---|---|---|---|---|---|---|---|---|---|---|---|---|---|---|---|---|
| *Without Age* | | | | | | | | | | | | | | | | | | | | |
|  |  | X |  |  |  |  |  |  |  | X |  |  |  |  | X |  |  | 0.954 | 0.772 | 0.768 |
| X |  | X |  |  |  |  |  |  |  | X |  |  |  |  | X |  |  | 0.965 | 0.757 | 0.754 |
|  | X | X |  |  |  |  |  |  |  | X |  |  |  |  | X |  |  | 0.956 | 0.752 | 0.759 |
|  |  | X |  |  | X |  |  |  |  | X |  |  |  |  | X |  |  | 0.950 | 0.770 | 0.753 |
|  |  | X |  |  |  | X |  |  |  | X |  |  |  |  | X |  |  | 0.952 | 0.760 | 0.754 |
|  |  | X |  |  |  |  |  |  |  | X | X |  |  |  | X |  |  | 0.954 | 0.828 | 0.757 |
|  |  | X |  |  |  |  |  |  |  | X |  |  |  |  | X | X |  | 0.960 | 0.768 | 0.767 |
| X |  |  |  |  |  |  |  |  |  | X |  |  |  |  | X | X |  | 0.961 | 0.771 | 0.756 |
|  |  |  | X |  |  |  |  |  |  | X |  |  |  |  | X | X |  | 0.898 | 0.754 | 0.778 |
| *With Age* | | | | | | | | | | | | | | | | | | | | |
|  |  | X |  | X |  |  |  |  |  | X | X |  |  |  | X |  |  | 0.961 | 0.823 | 0.791 |
|  |  | X |  | X |  |  |  |  |  | X |  | X |  |  | X |  |  | 0.963 | 0.752 | 0.778 |
|  |  | X |  | X |  |  |  |  |  | X |  |  |  |  | X | X |  | 0.970 | 0.764 | 0.789 |
| X |  |  |  | X |  |  |  |  |  | X |  |  |  |  | X | X |  | 0.977 | 0.764 | 0.764 |
|  | X |  |  | X |  |  |  |  |  | X |  |  |  |  | X | X |  | 0.909 | 0.758 | 0.752 |

These 14 panels, having at most 4 protein biomarkers, are found to provide AUC LOOCV >0.75 for all classifications, i.e. H/G vs P, H vs G, and MP vs AP:

14 protein markers are considered:

MMP8
IL-1β
MMP9
HGF
Free Light Chain (FLC) κ (kappa)
Free Light Chain (FLC) λ (lambda)
A1AGP
Hb-beta
Hb-delta
Keratin 4
Profilin
Pyruvate kinase
S100A8
S100A9

Furthermore, based on domain knowledge, in the logistic regression the additional predictors $\kappa+\lambda$, $\kappa-\lambda$, $\kappa/\lambda$ were considered.

With 14 protein markers, plus optionally age as marker, there are 2940 possible panels which have at most 4 protein markers (panel having only age is not considered). When considering the 3 extra predictors indicated above, there are 4204 non-redundant possible panels which have at most 4 protein markers (panel having only age is not considered). Non-redundant here means that a panel including e.g. $\kappa+\lambda$ and $\kappa-\lambda$, as predictors is not considered, as in the logistic regression it gives the same result as the corresponding panel including $\kappa$ and $\lambda$ as predictors. Not restricting number of protein markers in a panel, yields a number of 98302 non-redundant possible panels (panel having only age is not considered) considering the 14 protein markers, age, and mentioned additional 3 predictors.

While the invention has been illustrated and described in detail in the drawings and foregoing description, such illustration and description are to be considered illustrative or exemplary and not restrictive; the invention is not limited to the disclosed embodiments. For example, it is possible to present detection reagents for different biomarkers in different units. Or, conveniently, a kit of the invention can comprise a fixed set of detection reagents for the protein biomarkers that are used in all embodiments, i.e., MMP-9 and IL-1$\beta$, and flexible modules comprising a detection reagent for either of the further biomarkers, i.e., MMP-8 or MMP-3.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing whether a human patient has healthy periodontal tissue, has gingivitis, has mild periodontitis or has advanced periodontitis. The method is based on the insight to determine a selection of as few as three biomarker proteins. Accordingly, in a sample of saliva from a patient, the concentrations are measured of the proteins A1AGP, PK, and at least one of MMP-9 and S100A8. Based on the concentrations as measured, a value is determined reflecting the joint concentrations for said proteins. This value is typically used to calculate the probability of having gingivitis, periodontitis or advanced periodontitis, and compared with respective threshold values reflecting in the same manner the joint concentrations associated with gingivitis, periodontitis or advanced periodontitis. The comparison allows assessing whether the testing value is indicative of the presence of health, gingivitis, mild periodontitis or of advanced periodontitis in said patient.

**Claims**

1. An *in vitro* method for assessing whether a human patient is free from periodontal disease, has gingivitis, has mild periodontitis or has advanced periodontitis, wherein the method comprises:

   detecting, in a sample of saliva from said human patient, the concentrations of the proteins: Alpha-1-acid glycoprotein (A1AGP) and Pyruvate Kinase (PK), and at least one of the proteins Matrix metalloproteinase-9 (MMP-9) and S100 calcium binding protein A8 (S100A8);
   determining at least one testing value reflecting the joint concentrations determined for said proteins;
   so as to assess whether the testing value is indicative for freedom from periodontal disease, for gingivitis, for mild periodontitis or for advanced periodontitis in said patient.

2. An *in vitro* method according to claim 1, wherein a testing value is generated for one, two or three of (i) the probability of periodontitis versus health or gingivitis, (ii) the probability of gingivitis versus health, and (iii) the probability of advanced periodontitis versus mild periodontitis.

3. An *in vitro* method according to claim 1 or claim 2, comprising comparing said at least one testing value with at least one threshold value reflecting in the same manner the joint concentrations associated with gingivitis, periodontitis or advanced periodontitis.

4. A method according to claim 3, wherein the threshold value is based on the concentrations of the proteins in a set of samples, including samples from subjects with healthy gums, samples from subjects that have gingivitis, samples from subjects that have mild or moderate periodontitis and samples from subjects having advanced periodontitis.

5. A method according to any preceding claim, wherein the human patient is known to have periodontal disease and the method assesses whether the patient has gingivitis, mild periodontitis or has advanced periodontitis.

6. A method according to any preceding claim, wherein the age of the subject is determined and the testing value reflects the joint concentrations determined for said proteins in combination with the age of the subject.

7. A method according to any preceding claim, wherein the threshold value is based on the concentrations determined for the proteins in one or more reference samples each sample associated with the presence of healthy gums, gingivitis, mild periodontitis or advanced periodontitis.

8. A method according to any one of the preceding claims, wherein the proteins consist of A1AGP, PK, and one of MMP-9 and S100A8.

9. A method according to claim 8, wherein the proteins consist of: A1AGP, PK, and MMP-9; or A1AGP, PK, and S100A8.

10. A method according to any one of the preceding claims, wherein the concentration values determined are arithmetically processed into a number between 0 and 1.

11. The use of the proteins Alpha-1-acid glycoprotein (A1AGP), Pyruvate Kinase (PK), and at least one of the proteins Matrix metalloproteinase-9 (MMP-9) and S100 calcium binding protein A8 (S100A8) in a sample of saliva of a human patient, as biomarkers for assessing whether the patient is free from periodontal disease, has gingivitis, has mild periodontitis or has advanced periodontitis.

12. The use according to claim 11, wherein the age of the human patient is also used as a biomarker.

13. A system for assessing whether a human patient is free from periodontal disease, has gingivitis, has mild periodontitis or has advanced periodontitis, the system comprising:

    detection means able and adapted to detect in a sample of saliva of the human patient the proteins Alpha-1-acid glycoprotein (A1AGP) and Pyruvate Kinase (PK), and at least one of the proteins Matrix metalloproteinase-9 (MMP-9) and S100 calcium binding protein A8 (S100A8);
    a processor able and adapted to determine from the determined concentrations of said proteins an indication that the patient is free from periodontal disease, has gingivitis, has mild periodontitis or has advanced periodontitis.

14. A system according to claim 13, further comprising a container for receiving an oral fluid sample, the container comprising the detection means.

15. A system according to claim 13 or 14, further comprising:

    a user interface for presenting the indication to a user; and
    a data connection between the processor and the user interface for transferring the indication from the processor to the user interface.

16. A system according to any one of claims 13 to 15, wherein the processor is enabled to function by means of an internet-based application.

17. A system according to any of claims 13 to 16, wherein the interface is capable of putting in information on the age of the subject and the processor is able and adapted to determine from the determined concentrations of said proteins, an indication that the patient is free from periodontal disease, has gingivitis, has mild periodontitis or has advanced periodontitis.

18. A kit for detecting at least three biomarkers for periodontal disease in a sample of saliva of a human patient, said kit comprising one or more detection reagents for detecting Alpha-1-acid glycoprotein (A1AGP) and Pyruvate Kinase (PK), and at least one of the proteins Matrix metalloproteinase-9 (MMP-9) and S100 calcium binding protein A8 (S100A8).

19. A kit according to claim 18, wherein the one or more detecting reagents comprise at least three detection reagents, a first detection reagent for detecting A1AGP, a second detecting reagent for detecting PK, and a third detecting reagent for detecting one of MMP-9 and S100A8.

**20.** A kit according to claim 18 or 19, wherein the one or more detecting reagents are contained on a solid support.

**21.** A kit according to any one of the claims 18 to 20, wherein the one or more detecting reagents consist of detecting reagents for:

A1AGP, PK, and MMP-9, or
A1AGP, PK, and S100A8.

**22.** An *in vitro* method for determining a change in status of periodontal disease in a human patient over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins A1AGP, PK, and at least one of MMP-9 and S100A8, and comparing the concentrations, whereby a difference in any one, two, or all three of the concentrations, reflects a change in status.

**23.** A method of diagnosing whether a human patient is free from periodontal disease, has gingivitis, has mild periodontitis or has advanced periodontitis, comprising detecting in a sample of saliva of the human patient the proteins Alpha-1-acid glycoprotein (A1AGP) and Pyruvate Kinase (PK), and at least one of the proteins Matrix metalloproteinase-9 (MMP-9) and S100 calcium binding protein A8 (S100A8), and assessing the presence or absence of gingivitis, mild periodontitis or advanced periodontitis in the patient on the basis of the concentrations of said proteins in said sample.

**24.** A method of detecting the proteins Alpha-1-acid glycoprotein (A1AGP) and Pyruvate Kinase (PK), and at least one of the proteins Matrix metalloproteinase-9 (MMP-9) and S100 calcium binding protein A8 (S100A8) in a human patient suffering from gingivitis, mild periodontitis or advanced periodontitis, comprising:

(a) obtaining a saliva sample from a human patient; and
(b) detecting whether Alpha-1-acid glycoprotein (A1AGP) and Pyruvate Kinase (PK), and at least one of Matrix metalloproteinase-9 (MMP-9) and S100 calcium binding protein A8 (S 1 00A8) are present in the sample by contacting the sample with one or more detecting reagents for binding said proteins and detecting binding between each protein and the one or more detecting reagents.

FIG. 1

FIG. 2

FIG. 3

Europäisches
Patentamt

European
Patent Office

Office européen
des brevets

# EUROPEAN SEARCH REPORT

Application Number

EP 18 15 1827

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/037924 A2 (KONINKL PHILIPS NV [NL]) 13 March 2014 (2014-03-13) | 18,19,21 | INV. G01N33/68 |
| A | * claims 1-7 * * Supplementary Tables 1, 5, 11; table 4 * | 1-17,20, 22-24 | |
| A | IAN J. DAVIS ET AL: "Longitudinal quantification of the gingival crevicular fluid proteome during progression from gingivitis to periodontitis in a canine model", JOURNAL OF CLINICAL PERIODONTOLOGY, vol. 43, no. 7, 23 May 2016 (2016-05-23), pages 584-594, XP055454961, DK ISSN: 0303-6979, DOI: 10.1111/jcpe.12548 * Table S1; table 3 * * page 589, column 1, paragraph 2 - column 2, paragraph 1 * | 1-24 | |
| A | US 2011/020846 A1 (YU JAU-SONG [TW] ET AL) 27 January 2011 (2011-01-27) * paragraph [0023] * | 1-24 | TECHNICAL FIELDS SEARCHED (IPC) G01N |

The present search report has been drawn up for all claims

| Place of search | Date of completion of the search | Examiner |
|---|---|---|
| Munich | 28 February 2018 | Schwachtgen, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 1827

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2018

| Patent document cited in search report | Publication date | Patent family member(s) | Publication date |
|---|---|---|---|
| WO 2014037924 A2 | 13-03-2014 | CA 2884089 A1<br>CN 104620110 A<br>EP 2893353 A2<br>JP 2015529333 A<br>KR 20150052311 A<br>RU 2015113089 A<br>US 2015219665 A1<br>WO 2014037924 A2 | 13-03-2014<br>13-05-2015<br>15-07-2015<br>05-10-2015<br>13-05-2015<br>27-10-2016<br>06-08-2015<br>13-03-2014 |
| US 2011020846 A1 | 27-01-2011 | US 2011020846 A1<br>US 2011021750 A1 | 27-01-2011<br>27-01-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **RAMSEIER et al.** *J Periodontol.,* March 2009, vol. 80 (3), 436-46

- **HARLOW ; LANE.** Antibodies, A Laboratory Manual. 1988